# EUROPEAN PATENT SPECIFICATION

(11) **EP 3 993 715 B1**
(45) Date of publication and mention of the grant of the patent: **17.04.2024**
(21) Application number: 20734760.0
(22) Date of filing: 01.07.2020
(51) Int. Cl.: A61L 31/16, A61B 17/34, A61L 31/10, A61B 17/06, A61B 5/145, A61B 17/00, A61M 5/32, A61M 37/00

(54) **IMPLANTATION NEEDLE FOR INSERTING A SUBCUTANEOUSLY INSERTABLE ELEMENT INTO A BODY TISSUE**
IMPLANTATIONSNADEL ZUM EINSETZEN EINES SUBKUTAN EINFÜHRBAREN ELEMENTS IN EIN KÖRPERGEWEBE
AIGUILLE D'IMPLANTATION POUR INSERTION D'UN ÉLÉMENT INSÉRABLE TRANSCUTANÉMENT DANS UN TISSU CORPOREL

(30) Priority: 04.07.2019 EP 19184490
(43) Date of publication of application: 11.05.2022
(73) Proprietor: F. Hoffmann-La Roche AG, 4070 Basel (CH); Roche Diabetes Care GmbH, 68305 Mannheim (DE)
(72) Inventor: KUEBLER, Sebastian, 68305 Mannheim (DE)
(74) Representative: Kierig, Elisabeth
(86) International application number: PCT/EP2020/068448
(87) International publication number: WO 2021/001394

(56) References cited:
- WO-A2-2006/053007
- US-A1- 2010 324 579
- US-A1- 2018 200 412

## Description

### Technical Field

The present invention relates to an implantation needle for inserting a subcutaneously insertable element into a body tissue. The present invention further relates to a kit for inserting a subcutaneously insertable element into a body tissue. Furthermore, the present invention relates to a method of manufacturing an implantation needle. The devices and the method according to the present invention may mainly be used for delivering insulin to a user or a patient and/or for conducting at least one analytical measurement of a body fluid of the user or the patient. The invention may both be applied in the field of home care as well as in the field of professional care, such as in hospitals. Other applications are generally feasible.

### Background art

Immunosuppressive pharmaceutical compounds have a very broad spectrum of medical uses, from self-medication in treatment of allergic coryza to suppression of rejection in graft recipients. Immunosuppressive compounds are often provided as immediate release formulations, however, in some applications sustained-release formulations may be preferred. E.g. in the field of ophthalmology, corticosteroids have been used for the treatment of macular edema. As an intravitreal delivery system, a pen-like instrument was proposed, delivering a dose of a small, biodegradable and sustained-release formulation of dexamethasone (Meyer et al. (2012), Retina 32:2133; Fialho & da Silva Cunha (2005), Drug Delivery 12(2): 109).

For inserting implantable elements, e.g. implantable sensors, under the skin of a subject, a variety of implantation needles are known, e.g. closed cannulas with a V-bevel, oval-shaped slotted cannulas with a V-bevel, slotted cylindrical cannulas, U-shaped cannulas with a rectangular cross-section, or peel catheters, i.e. a cannula tube divided into two with a V-bevel which is then opened in the skin and removed in separate parts; e.g. as described in WO 2015/128263 A1, DE 10 2011 112021 A1, DE 102 24 101 A1, WO 99/53991 A1, US 2010/ 324579, US 3,064,651, US 3,448,740, WO 2005/044116, and US 4,490,139.

A common problem with implantation of implantable elements is that the implantable elements may cause a defense reaction of the body of a subject to occur, which may lead to inflammation, scarring, and other immune responses to the material of the implantable element. This may lead to a limitation of the application period of subcutaneous continuous glucose monitoring sensors. Further, an encapsulation may take place when applying insulin infusion kits. For this reason, use of pharmaceutical compounds improving tolerance has been proposed, in particular topical administration at the site of implantation (cf., e.g. CA 2 664 426; WO 2006/055008). Moreover, implantable elements, in particular implantable sensors like continuous glucose measurement (CGM) sensors, have been covered with membranes comprising pharmaceutical compounds improving tolerance to suppress local immune reactions (cf., e.g. W.M. Reichert et al, Acta Biomaterialia, 30(2016), 106-115). Nonetheless, immune reactions and rejection of implantable elements cannot always be avoided. Moreover, manufacturing of coated implantable elements is rather complex and, therefore, cost-intensive.

EP 3 060 273 A1 describes methods and devices for reducing a diabetic patient's foreign body immune response, including infusion site-loss and/or occlusion. Such foreign body responses are associated with the treatment of the diabetic patient where the treatment requires subcutaneous implantation of a foreign body, such as a cannula or catheter. In certain embodiments of the invention, a response-inhibiting agent is administered to a patient at the site of cannula/catheter insertion, thereby facilitating delivery of insulin to the diabetic patient and mitigating site-loss and/or occlusion over a period of time.

US 2018/0199886 A1 describes a sensor device for measuring an analyte concentration in a host. The sensor device comprises a sensor unit comprising a sensor body, at least one electrode, and a membrane covering at least a portion of the at least one electrode. Further, the sensor device comprises a tissue-piercing element comprising a dissolvable material that dissolves after insertion into the host, the tissue-piercing element abutting the sensor body and being capable of piercing tissue. Further, the sensor device comprises a mounting unit spaced from the sensor tip and configured to support the sensor device on an exterior surface of the host's skin.

Despite the advantages of state of the art devices and methods several technical challenges remain. Specifically, a manufacturing of the implantation needle and of the subcutaneously insertable element is time-consuming and expensive.

### Problem to be solved

It is therefore an objective of the present invention to provide an implantation needle for inserting a subcutaneously insertable element into a body tissue, a kit for inserting a subcutaneously insertable element into a body tissue and a method of manufacturing an implantation needle which at least partially avoid the shortcomings of known devices and methods of this kind and which at least partially address the above-mentioned challenges. Specifically, devices and methods shall be disclosed which provide improved means and methods for preventing immune reactions to implantable elements. Further, devices and methods shall be disclosed which allow for an easy manufacturing and simple handling processes by a user or a patient.

### Summary

This problem is addressed by an implantation needle for inserting a subcutaneously insertable element into a body tissue as defined in claim 1, a kit for inserting a subcutaneously insertable element into a body as defined in claim 11, and a method of manufacturing an implantation needle as defined in claim 12. In the embodiments according to the present invention, the pharmaceutical compound is configured to remain at least partially in the body tissue after insertion of the subcutaneously insertable element (112), wherein the pharmaceutical compound is configured to be released via a mechanical stripping process during withdrawal of the implantation needle (110) from the body tissue or via a dissolving process of the pharmaceutical compound.

As used in the following, the terms "have", "comprise" or "include" or any arbitrary grammatical variations thereof are used in a non-exclusive way. Thus, these terms may both refer to a situation in which, besides the feature introduced by these terms, no further features are present in the entity described in this context and to a situation in which one or more further features are present. As an example, the expressions "A has B", "A comprises B" and "A includes B" may both refer to a situation in which, besides B, no other element is present in A (i.e. a situation in which A solely and exclusively consists of B) and to a situation in which, besides B, one or more further elements are present in entity A, such as element C, elements C and D or even further elements.

Further, it shall be noted that the terms "at least one", "one or more" or similar expressions indicating that a feature or element may be present once or more than once typically will be used only once when introducing the respective feature or element. In the following, in most cases, when referring to the respective feature or element, the expressions "at least one" or "one or more" will not be repeated, non-withstanding the fact that the respective feature or element may be present once or more than once.

Further, as used in the following, the terms "preferably", "more preferably", "particularly", "more particularly", "specifically", "more specifically" or similar terms are used in conjunction with optional features, without restricting alternative possibilities. Thus, features introduced by these terms are optional features and are not intended to restrict the scope of the claims in any way. The invention may, as the skilled person will recognize, be performed by using alternative features. Similarly, features introduced by "in an embodiment of the invention" or similar expressions are intended to be optional features, without any restriction regarding alternative embodiments of the invention, without any restrictions regarding the scope of the invention and without any restriction regarding the possibility of combining the features introduced in such way with other optional or non-optional features of the invention.

As generally used within the present invention, the terms "patient" and "user" may refer to a human being or an animal, independent from the fact that the human being or animal, respectively, may be in a healthy condition or may suffer from one or more diseases. As an example, the patient or the user may be a human being or an animal suffering from diabetes. However, additionally or alternatively, the invention may be applied to other types of users or patients or diseases.

The term "body tissue" as used herein is a broad term and is to be given its ordinary and customary meaning to a person of ordinary skill in the art and is not to be limited to a special or customized meaning. The term specifically may refer, without limitation, to a cellular organizational level intermediate between cells and a complete origin. The body tissue may specifically be an ensemble of similar cells from the same that together carry out a specific function. Thereby, organs may then be formed by functional grouping together of multiple tissues. As an example for body tissue, interstitial tissue, i.e. connective tissue between cellular elements if a structure, may be named. As further used herein, the term "body fluid" generally may refer to a fluid which is typically present in a body or the body tissue of the user or the patient and/or which may be produced by the body of the user or the patient.

Thus, as an example, the body fluid may be selected from the group consisting of blood and interstitial fluid. However, additionally or alternatively, one or more other types of body fluids may be used, such as saliva, tear fluid, urine or other body fluids.

In a first aspect of the present invention an implantation needle for inserting a subcutaneously insertable element into a body tissue as defined in claim 1 is provided. The implantation needle comprises at least one covering comprising at least one pharmaceutical compound. The pharmaceutical compound is configured to at least partially remain in the body tissue after insertion of the subcutaneously insertable element. Specifically, the pharmaceutical compound may be configured to be released at least partially into the body tissue after insertion of the subcutaneously insertable element.

The term "implantation needle" as used herein is a broad term and is to be given its ordinary and customary meaning to a person of ordinary skill in the art and is not to be limited to a special or customized meaning. The term specifically may refer, without limitation, to an arbitrary compartment which may be implantable or insertable at least partially into an arbitrary body tissue, particularly in order to deliver or to transfer a further element. Therefore, the implantation needle may specifically be or may comprise a hollow tube or a hollow needle. Thus, the implantation needle may also be referred to as implantation cannula or as insertion needle. In particular, the implantation needle is removed after the further element is delivered or transferred. Thus, preferred is an implantation needle for inserting a subcutaneously insertable element into a body tissue, wherein the implantation needle is withdrawn from the body tissue after, preferable immediately after insertion of the subcutaneously insertable element.

The implantation needle may have a lumen which is fully or partially enclosed by a wall of the insertion cannula. The term "lumen" generally refers to an interior volume of an arbitrary element. The interior volume may specifically be an open interior volume. Thus, the interior volume may not be fully enclosed or surrounded by a wall of the element. Instead, a flow of a fluid medium or an insertion of another object from one end of the element to a further end through the lumen may be feasible. As further used herein, the term "wall" may generally refer to an arbitrary structure, specifically a structural material, which is configured to at least partially surround another object or volume thereby defining physical limits of an object. Further, the wall may be configured to protect the volume or the other object at least partially enclosed by the wall.

The implantation needle specifically may have an elongated shape and may be configured to pierce a solid or semisolid surface, in particular the skin of the user or the patient, and adapted for implanting the subcutaneously insertable element at least partially into the body tissue, specifically under the skin of the user or the patient.

The implantation needle may comprise a subcutaneously insertable element receiving portion. As used herein, the term "subcutaneously insertable element receiving portion" relates to a section of the implantation needle adapted for receiving the subcutaneously insertable element as specified elsewhere herein. The subcutaneously insertable element receiving portion may correspond to the lumen such as described above or may be a section of the lumen such as described above. Specifically, the subcutaneously insertable element receiving portion may be a proximal receiving portion, the term "proximal" being used herein relative to an operator, specifically to the user or the patient, of the implantation needle. Thus, specifically, the subcutaneously insertable element receiving portion may have dimensions suitable for receiving the subcutaneously insertable element. Exemplarily, the subcutaneously insertable element receiving portion may have a largest inner diameter of from 0.25 mm to 1.5 mm, preferably from 0.5 mm to 1 mm, preferably of about 0.65 mm, preferably of 0.67 mm. Specifically, the subcutaneously insertable element receiving portion may have a length of from 1 mm to 15 mm, more specifically of from 5 mm to 12 mm.

Further, the implantation needle may have a tip portion. The tip portion may be configured to puncture or to pierce the skin of the patient or the user. For this purpose, the tip portion may have a slot extending in an axial direction of the implantation needle. The term "slot" may generally refer to an opening or a slit. Specifically, the slot may be located at an end of the tip portion. Thereby, the slot may form an angle of 10° to 80°, more preferably of 20° to 60°, to a longitudinal axis of the implantation needle. The slot may be configured to facilitate an insertion of the implantation needle into the body tissue. The tip portion may be the most distal portion of the implantation needle, the term "distal" being used herein relative to the operator, specifically to the user or the patient, of the implantation needle.

The tip portion may correspond to an in vivo distal end of the implantation needle. As further used herein, the term "in vivo distal end" may refer to a part, specifically to an end of an object which is configured to be at least partially insertable into a living organism, specifically into a body tissue. Therefore, the in vivo distal end may refer to an end opposing an end which corresponds to a point of attachment of the object. On the contrary, the term "ex vivo proximal end" may refer to a part, specifically to an end of an obj ect which is configured to stay outside of a living organism. Therefore, the ex vivo proximal end may refer to an end which corresponds to a point of attachment of the object. Thus, the in vivo distal end and the ex vivo proximal end may be opposing ends of one single object, whereby the object is configured to be partially inserted into a living organism via the in vivo distal end while one part of the object may stay outside of the living organism.

The implantation needle may comprise or may consist of a rigid or semi-rigid material. Specifically, the rigid or semi-rigid material may be a metal, a plastic, or a ceramic. More specifically, the implantation needle may comprise or may consist of steel such as stainless steel. The implantation needle may specifically at least partially be made of at least one biocompatible material, i.e. a surface which, at least during durations of use, does not have any detrimental effects on the user, the patient or the body tissue.

Specifically, the implantation needle may comprise the lumen, specifically, at least one inner lumen completely or partially enclosed laterally by the rigid or semi-rigid material of the implantation needle. Specifically, the implantation needle may be an open implantation needle, e.g. a slotted implantation needle as specified herein below or the implantation needle may be a closed implantation needle, i.e., the rigid or semi-rigid material completely encloses the inner lumen along the elongated axis. Further, a cross-section of the inner lumen of the implantation needle may be essentially round, in particular circular or oval, V-shaped, or U-shaped. In an embodiment, a cross-section of the inner lumen has the same shape over at least ½, in a further embodiment 2/3 of needle length, in an embodiment has essentially the same shape from the proximal end of the tip portion to the proximal end of the subcutaneously insertable element receiving portion. In an embodiment, a cross-section of the inner lumen of the implantation needle has an essentially round shape; in such case, in an embodiment, the largest inner diameter of the implantation needle is of from 0.25 mm to 1.5 mm, in a further embodiment of from 0.5 to 1 mm, in a further embodiment of about 0.65 mm, in a further embodiment of 0.67 mm. In an embodiment, the largest inner diameter is essentially constant over at least ½, in a further embodiment 2/3 of implantation needle length, in an embodiment is constant at least from the proximal end of the tip portion to the proximal end of the subcutaneously insertable element receiving portion. In an embodiment, the cross-section of the inner lumen of the implantation needle has a U or V shape; in such case, the above values for a largest inner diameter apply to the largest distance of the lateral walls mutatis mutandis. As will be understood by the skilled person, also mixed open/closed embodiments are envisaged, e.g. implantation needles closed at the subcutaneously insertable element receiving portion, but open at the tip portion. In an embodiment, the implantation needle is an implantation needle essentially as disclosed by WO 2018/166963.

Thus, specifically, the implantation needle may comprise the subcutaneously insertable element receiving portion and the tip portion, specifically a slant tip portion. The slant tip portion, in an embodiment, further comprises: a first slant surface contiguous to a first outer peripheral surface of an implantation needle main body, wherein the first slant surface is provided as a first non-cutting edge; a second slant surface contiguous to a second outer peripheral surface of a hollow needle main body, wherein the second slant surface is provided as a second non-cutting edge; and a pair of sharpened surfaces symmetric with respect to an edge point and a longitudinal axis of the implantation needle main body, wherein the sharpened surfaces are both provided with a cutting edge. The first slant surface comprises a first flank, and the second slant surface comprises a second flank. The first flank is provided at a first distance from the edge point, and the second flank is provided at a second distance from the edge point which is different from the first distance.

The implantation needle main body may be an open implantation needle main body. In other words, in an embodiment, the implantation needle main body does not have a closed tube shape. The opening in the side of the implantation needle main body may extend along the entire length of the implantation needle main body. Alternatively, the opening in the side of the implantation needle main body may only extend along part of the length of the implantation needle main body. In case the opening extends only along part of the length of the implantation needle main body, the opening, in an embodiment, extends to a distal end of the implantation needle main body, providing an opening towards the slant tip portion. In an embodiment, the opening in the side of the implantation needle main body may be formed symmetric with respect to the longitudinal axis of the implantation needle main body. The opening in the side of the implantation needle main body may be provided as a slot opening. Thereby, a slotted implantation needle main body may be provided. Inner edges formed in the range of the opening in the side of the implantation needle main body may be provided as non-cutting edges.

In the following it is described how a needle element may be manufactured. The needle element may be configured to be covered with the pharmaceutical compound as described above or as will further be described below in more detail such that the implantation needle is formed. The method may comprise a) punching a flat metal strip or sheet so as to give rise to a flat sheet of a desired shape suitable for later bending the sheet so as to give rise to the shape of the needle element. In a second step b) the sheet may then be subjected to embossing of "dull" non-cutting edges in a portion of the sheet. Then, in a step c) a needle element main body and slant surfaces of a tip portion of the needle element may be bent and the tip portion may be embossed and punched out so as to give rise to the needle element. As an alternative, etching methods can be used to create a sharp tip of the needle element. In a further step d), a fixing element, in particular a tongue, may be punched out. As will be understood by the skilled person, step d) may also be performed concomitantly to step a). The method for manufacturing the needle element may comprise producing at least first and second flanks of the needle element by at least one of a punch-bent process or an etching process combined with a bent process. The punch-bent process combining punching and bending the material used for manufacturing the needle element are combined for producing at least one of the flanks. Such punch-bent process may be used for manufacturing the needle element main body as well.

The term "insertable element" as used herein is a broad term and is to be given its ordinary and customary meaning to a person of ordinary skill in the art and is not to be limited to a special or customized meaning. The term specifically may refer, without limitation, to an arbitrary element which may be configured to be at least partially insertable into another object such that the insertable element may be situated at least partially located under the object or surrounded by an interior of the object. Specifically, the insertable element may be configured to be at least partially inserted into the body tissue, specifically under the skin of the patient. Therefore, the insertable element may specifically have an elongate shape with a small cross-section. Insertable elements are, in principle, known in the art. The term "subcutaneously insertable element" as further used herein is a broad term and is to be given its ordinary and customary meaning to a person of ordinary skill in the art and is not to be limited to a special or customized meaning. The term specifically may refer, without limitation, to a property of an insertable element of being adapted to be fully or at least partly arranged within a body tissue or under a skin site of a patient or a user. For this purpose, the subcutaneously insertable element may comprise a subcutaneously insertable portion.

Exemplarily, the subcutaneously insertable element may be selected from the group consisting of: a sensor, an infusion cannula, a drainage tubing, an intravascular catheter, a temporary pacemaker electrode, an oxygenator, an extracorporeal oxygenator tubing and accessories, a dialyzer, a dialysis tubing and accessories, a dosing tube. However, also other embodiments of the subcutaneously insertable element may be feasible.

Specifically, the subcutaneously insertable element may be a sensor, specifically a biosensor, preferably an analyte sensor for detecting at least one analyte in a body fluid. Specifically, the sensor may be configured for remaining under the skin after removing the implantation needle. The sensor may be fully or partially implantable into the body tissue. As further used herein, the terms "sensor" and "analyte sensor" may generally refer to an arbitrary element which is adapted to perform a process of detection and/or which is adapted to be used in the process of detection. Thus, the sensor specifically may be adapted to determine the concentration of the analyte and/or a presence of the analyte. The term "detection" generally refers to a process of determining a presence and/or a quantity and/or a concentration of the at least one analyte. Thus, the detection may be or may comprise a qualitative detection, simply determining the presence of the at least one analyte or the absence of the at least one analyte, and/or may be or may comprise a quantitative detection, which determines the quantity and/or the concentration of the at least one analyte. As a result of the detection, at least one signal may be produced which characterizes an outcome of the detection, such as at least one measurement signal. The at least one signal specifically may be or may comprise at least one electronic signal such as at least one voltage and/or at least one current. The at least one signal may be or may comprise at least one analogue signal and/or may be or may comprise at least one digital signal. The analyte sensor specifically may be an electrochemical sensor. The sensor may specifically be a glucose sensor.

As outlined above, the subcutaneously insertable element may be an infusion cannula. The term "infusion cannula" may generally refer to an arbitrary cannula being configured to introduce an infusion, i.e. a liquid substance, specifically a liquid substance comprising a medicine, into the body tissue. Therefore, the infusion cannula may be attached to a reservoir comprising the liquid substance, specifically via the ex vivo proximal end of the infusion cannula. The infusion cannula may be part of an infusion kit. The term "infusion kit" may refer to an assembly of components which are required for a conduction of an arbitrary infusion. Thus, besides of the infusion cannula, the infusion kit may further comprise at least one fluid coupling for coupling the infusion kit to at least one medication device, preferably to at least one medication pump.

The subcutaneously insertable element may have an elongated or round form. Specifically, the subcutaneously insertable element may have the form of one of a cylinder, rounded cylinder. Further, the subcutaneously insertable element may have a rectangular cross section or a trapezoidal cross-section. The subcutaneously insertable element may have an outer diameter from 0.25 mm to 1.5 mm, preferably from 0.5 mm to 1 mm, preferably of about 0.65 mm, more preferably of 0.67 mm. As will be understood by the skilled person, an outer diameter of the subcutaneously insertable element may be smaller than an inner diameter of the implantation needle. Specifically, the outer diameter of the subcutaneously insertable element may be up to 80%, preferably up to 90%, preferably up to 95%, preferably up to 99% of the inner diameter of the implantation needle, specifically of the subcutaneously insertable element receiving portion of the implantation needle. As will be further understood by the skilled person, the above applies mutatis mutandis to subcutaneously insertable elements having a cross-section profile with a limited number of symmetry axes, e.g. only one axis of symmetry, and to irregularly shaped subcutaneously insertable elements.

In an embodiment, the subcutaneously insertable element does not comprise the at least one pharmaceutical compound which the implantation needle comprises. In particular, the subcutaneously insertable element does not comprise a covering of the at least one pharmaceutical compound.

The term "insertion" as further used herein is a broad term and is to be given its ordinary and customary meaning to a person of ordinary skill in the art and is not to be limited to a special or customized meaning. The term specifically may refer, without limitation, to an arbitrary progress of placing an element at least partially, e.g. fully or partially, within another object, specifically through at least one surface of the other object. Thus, after the progress of insertion, the element may be partially located within the other object and may specifically penetrate the other object and may further be partially located outside of the other object. However, alternatively, the element may be fully located within the other object, specifically the element may be fully located in an interior space of the other object. Specifically, the insertion may correspond to a subcutaneous insertion or a subcutaneous implantation.

The term "subcutaneous" as further used herein is a broad term and is to be given its ordinary and customary meaning to a person of ordinary skill in the art and is not to be limited to a special or customized meaning. The term specifically may refer, without limitation, to a property of an arbitrary element of being adapted to be fully or at least partly arranged within a body tissue or under a skin site of a patient or a user. For this purpose, the element may comprise an insertable portion. In order to further render the element to be usable as a subcutaneous element, the element may fully or partially provide a biocompatible surface, i.e. a surface which, at least during durations of use, do not have any detrimental effects on the user, the patient or the body tissue. Further, the subcutaneous insertable element generally may be dimensioned such that an insertion of the element into the body tissue is feasible, such as by providing a width in a direction perpendicular to an insertion direction of no more than 5 mm, preferably of no more than 2 mm, more preferably of no more than 1.5 mm. Specifically, the object may be configured to be introduced under the skin, exemplarily as an injection.

The term "covering" as further used herein is a broad term and is to be given its ordinary and customary meaning to a person of ordinary skill in the art and is not to be limited to a special or customized meaning. The term specifically may refer, without limitation, to a material which is deposited on a surface of an arbitrary element or to a process of depositing a material on a surface of an arbitrary element. The covering may specifically be a coating. However, other embodiments may be feasible such as a deposition of a material by a dispense process. The covering may cover the object completely or may only cover a part or parts of the object. The covering may specifically be applied via a covering process, specifically a coating process, wherein a material is provided as a fluid medium and the fluid medium may be distributed on the surface. The covering may be a coating covering the object completely or may cover only part of the object. However, other embodiments may also be feasible. Thus, the material may also be provided as a solid such as a powder. Exemplarily, the coating process being configured to depositing the covering may be or may comprise the spray coating as described above or as will further be described below in more detail. The covering may specifically have a lateral extension exceeding its thickness by at least a factor of 2, at least a factor of 5, at least a factor of 10, or even at least a factor of 20 or more. Exemplarily, covering may have a thickness of 1 nm to 200 µm, preferably of 10 nm to 100 µm. In case the pharmaceutical compound is a highly potent substance the coating may specifically have a thickness of 1 nm to 50 nm, preferably of 10 nm to 30 nm. In case the pharmaceutical compound is embedded in a matrix the coating may specifically have a thickness of 1 µm to 200 µm, preferably of 100 µm to 150 µm. Specifically, the coating may be formed as a continuous coating. Thereby, the coating may be formed as one unit wherein the coating is at least to a large extent free from interruptions.

The term "pharmaceutical compound" as further used herein is a broad term and is to be given its ordinary and customary meaning to a person of ordinary skill in the art and is not to be limited to a special or customized meaning. The term specifically may refer, without limitation, to an arbitrary drug used to cure, treat or prevent disease. Thus, the pharmaceutical compound may also be referred to as medication, medicine, drug or as pharmaceutical drug. Specifically, the pharmaceutical compound may be selected from the group consisting of: an immunosuppressive, specifically a glucocorticoid immunosuppressive agent, an anti-allergic, an anti-inflammatory agent. The term "immunosuppressive" may refer to an arbitrary compound which is configured to reduce an activation or efficacy of an immune system of a patient. The term "anti-allergic" may refer to an arbitrary compound which is configured to reduce an allergic disease caused by hypersensitivity of the immune system to typically harmless substances in an environment of a patient. The term "anti-inflammatory agent" may refer to an arbitrary substance which is configured to reduce inflammation or swelling.

The pharmaceutical compound may comprise at least one compound which is configured to improve a tolerance of the subcutaneously insertable element in a body of the user or the patient, specifically at a site of implantation. The term "improving tolerance" may specifically comprise at least one process selected from the group consisting of: reducing immune rejection, reducing scarring, reducing growth of infectious agents. Appropriate pharmaceutical compounds for improving tolerance are, in principle, known in the art. Specifically, improving tolerance is locally improving tolerance at a site of implantation, more specifically at a site of subcutaneous implantation.

The pharmaceutical compound may specifically be selected from the group consisting of: dexamethasone or betamethasone or a salt, derivative, and/or prodrug thereof, a mixture of dexamethasone and betamethasone. The pharmaceutical compound may be Betamethasone, which has the IUPAC-name (8S,9R,10S,11S,13S,14S,16S,17R)-9-Fluoro-11,17-dihydroxy-17-(2-hydroxy acetyl)-10,13,16-trimethyl-6,7,8,11,12,14,15,16-octahydro-cyclo-penta[a]phenanthren-3-one, (CAS number 378-44-9). The pharmaceutical compound may be Dexamethasone, which has the IUPAC-name (8S,9R,10S,11S,13S,14S,16R,17R)-9-Fluoro-11,17-dihydroxy-17-(2-hydroxyacetyl)-10,13,16-trimethyl-6,7,8,11,12,14,15,16-oc-tahydro-cyclopenta[a]phenanthren-3-one, (CAS number 50-02-2).

In an embodiment of the present invention, the term "derivative" means a compound that can be derived from another compound by a chemical reaction, in particular by replacing an atom or a group of the compound by another atom or another group via chemical reaction.

In an embodiment, the derivative of Betamethasone or Dexamethasone is selected from the group consisting of 17-Oxo Betamethasone, Betamethasone hydrochloride, Betamethasone 21-acetate, Betamethasone 17-propionate, Betamethasone 21 propionate, Betamethasone phosphate, Betamethasone 17-valerate, Betamethasone 21-valerate, Betamethasone 17-benzoate, Betamethasone 17,21,-dipropionate, Betamethasone acibutate, Betamethasone sodium phosphate, Betamethasone butyrate propionate, Betamethasone tripropionate, 3-Hydroxy dexamethasone, 6 Hydroxydexamethasone, dexamethasone 17-acetate, dexamethasone 17-carboxamide, dexamethasone 17-propionate, dexamethasone 21-valerate, dexamethasone 21-acetate, dexamethasone 21-beta-D-glucoside, dexamethasone 21-linolate, dexamethasone 21-mesylate, dexamethasone 21-O-b-D-galactopyranose, dexamethasone 21-palmitate, dexamethasone 21-propionate, dexamethasone 21-sodium hydrogen phosphate, dexamethasone 21-sulfate, dexamethasone 21-tributylacetate, dexamethasone acefurate, dexamethasone 17-carboxylic acid, dexamethasone beta-D-glucuronide, dexamethasone dipropionate, Dexamethasone hemisuccinate, dexamethasone laurate, dexamethasone phosphate, dexamethasone sodium phosphate, dexamethasone tridecylate, dexamethasone valerate, dexamethasone 21-isonicotinate; in an embodiment is selected from the list consisting of Betamethasone 21-acetate, Betamethasone 17-propionate, Betamethasone 21 propionate, Betamethasone 17-valerate, Betamethasone 21-valerate, Betamethasone butyrate propionate, Betamethasone tripropionate, dexamethasone 17-acetate, dexamethasone 17-carboxamide, dexamethasone 17-propionate, dexamethasone 21-valerate, dexamethasone 21-acetate, dexamethasone 21-linolate, dexamethasone 21-mesylate, dexamethasone 21-palmitate, dexamethasone 21-propionate, dexamethasone 21-tributylacetate, dexamethasone acefurate, dexamethasone dipropionate, dexamethasone laurate, dexamethasone tridecylate, dexamethasone valerate.

Further, specifically, the pharmaceutical compound may further comprise pharmaceutically active compounds, in particular further anti-inflammatory, anti-allergic, and/or immunosuppressive compounds.

The pharmaceutical compound may be configured to increase the biocompatibility of the subcutaneously insertable element. The term "biocompatible", as used herein, is a broad term and is to be given its ordinary and customary meaning to a person of ordinary skill in the art and is not to be limited to a special or customized meaning. Specifically, the term may refer to a property of a compound or device, in particular an implantable or subcutaneously insertable element as specified elsewhere herein, of not being deleterious to a living system, in an embodiment a subject, contacted therewith. Specifically, the term relates to biocompatibility according to the IUPAC definition, i.e. the ability of a compound or device, in particular an implantable element as specified elsewhere herein, to be in contact with a living system, in an embodiment a subject, without producing an adverse effect or by producing a minimally adverse effect. Long-term minimally-invasive biosensors commonly lead to foreign body responses such as biofouling, fibrous capsule formation and inflammation. The use of small anti-inflammatory and/or antigenic molecules, such as dexamethasone, to influence tissue integration has been intensively studied. Here, the drug delivery system with biocompatible and controllable particles, like poly(lactic-co-glycolic acid) is pursued to limit inflammation and take advantage of the wound healing properties of this material (Current and Emerging Technology for Continuous Glucose Monitoring; Sensors 2017, 17, 182; doi: 10.3390/s17010182).

The covering may comprise at least one excipient. The term "excipient" is known to the skilled person to relate to a pharmaceutically inactive carrier compound in a pharmaceutical formulation being compatible with the other ingredients of the formulation and being not deleterious to the recipient thereof. Specifically, the excipient may be biocompatible as specified herein above. Further, the excipient may be biodegradable as specified herein below. Specifically, the excipient may be biocompatible and biodegradable.

The excipient may be selected from the group consisting of (i) polylactides, (ii) polygly-colides, (iii) polydioxanones, (iv) combinations of at least two of (i) to (ii), and (v) copolymers of monomers of at least two of (i) to (ii). (i), (ii) and (iv) are preferred. As is understood by the skilled person, the term "combination" in the context of the excipients of the present invention includes mixtures as well as non-mixed combinations, such as layered combinations. The term "copolymers" includes statistical as well as block copolymers. In an embodiment, the excipient comprises, in an embodiment consists of, a polylactide or a poly(lactide-co-glycolide), in a further embodiment a poly(lactide-co-glycolide) 50/50.

The term "polylactide" relates to a polymer based on lactic acid or a derivative thereof as a monomer. Polylactides are polyesters and are also referred to as polylactic acids. Specifically, the polylactide may be poly(lactic acid), CAS number 26100-51-6. The term "polyglycolide" may relate to a polymer based on glycolic acid or a derivative thereof as a monomer. The derivative may be selected from the group consisting of: Poly(D,L-lactide-co-glycolide) having a relation of the polymer units of 50:50, Mw 7000-69000 g/mol, end group: ester, acid; degradation time frame < 3 months; polylactic acids, Mw 2500-200000 g/mol, end group: ester, acid, degradation time frame < 3 months; Poly[(R)-3-hydroxybutyric acid], Mw 2500-200000 g/mol, degradation time frame < 1 months; Poly(3-hydroxybutyric acid-co-3-hydroxyvaleric acid; 0-100%; Mw 2500-200000 g/mol; degradation time frame < 1 months. The Mw may be determined by GPC according to Journal of Polymer Science: Polymer Chemistry Edition, Vol. 21, No. 1, 1983, p. 197-208. Specifically, the polyglycolide may be poly(glycolic acid), CAS number 26009-03-0. The term "polydioxanone" may relate to a polymer based on p-dioxanone or a derivative thereof as a monomer. Further, specifically, the polydioxanone is poly(p-dioxanone), CAS number 31621-87-1.

As outlined above, the excipient may be biodegradable. Specifically, the excipient may be a biodegradable polymer. The term "biodegradable", as used herein, relates to the property of a compound of being degradable by a living system, in particular a subject. Specifically, the term may relate to biodegradability according to the IUPAC definition, i.e. to a compound susceptible to degradation by biological activity, with the degradation accompanied by a lowering of its molecular mass. Specifically, said biodegradability may be biodegradability within a subject, specifically after subcutaneous implantation into a subject. Further, specifically, biodegradability may require a degradation half-life of a compound of at most 30 days after subcutaneous implantation in a mammal, in an embodiment a mouse. A degradation time may be tunable due to different compositions, surface characteristics, molecular weight, water content, glass transition temperature. Exemplarily, the excipient may have a degradation time of 5 of 50 days, preferably of 10 to 40 days, more preferably of 15 to 35 days and most preferably of 25 to 30 days. Reference is made to Poly Lactic-co-Glycolic Acid (PLGA) as Biodegradable Controlled Drug Delivery Carrier; Polymers (Basel). 2011 September 1; 3(3): 1377-1397. The degradation time may be dependent on the application. In case the implantation needle is applied in a continuous glucose monitoring device which commonly may have a wear time of 14 to 30 days, the degradation time may specifically be 2 to 25 days.

A degradation half-life may be determined after subcutaneous implantation of a spherical mass of 0.1 g of the compound. For every compound class a different method is commonly needed. A method for Poly(D,L-lactide-co-glycolide) is described under: Degradation of Poly(D,L-lactide-co-glycolide) 50:50 Implant in Aqueous Medium Iranian Polymer Journal 14 (8), 2005, 753-763.

Further, the pharmaceutical compound may be formulated in combination with other drugs either in a common pharmaceutical composition or as separated pharmaceutical compositions. The separated pharmaceutical composition may be provided in form of, e.g. a coating on the implantation needle.

Specifically, the pharmaceutical compound may be dexamethasone and the covering may comprise of from 100 µg to 5 mg dexamethasone, preferably of from 200 µg to 2 mg dexamethasone, preferably of from 500 µg to 1.5 mg dexamethasone. Further, the covering may comprise of from 100 µg to 5 mg excipient, preferably of from 200 µg to 2 mg excipient, more preferably of from 500 µg to 1.5 mg excipient. The values may be dependent from a choice of the pharmaceutical compound.

The covering may cover different sections of the implantation needle. Specifically, the covering may cover a surface section of the implantation needle. More specifically, the covering may cover a surface section of an insertable area of the implantation needle. As further used herein, the term "surface section" may refer to a part, specifically to a distinct part, of a surface. As further used herein, the term "insertable area" may refer to an area of the implantation needle, which belongs to a portion of the implantation needle which is configured to be inserted into the body tissue of the patient. Exemplarily, the term surface section may refer to at least 5%, preferably at least 10%, preferably at least 20%, preferably at least 30%, preferably at least 40%, preferably at least 50%, preferably at least 60%, preferably at least 70%, preferably at least 80%, preferably at least 90%, preferably at least 95% of the insertable area. However, other embodiments may be feasible. Further, the term surface section may refer to a whole surface of the insertable area, e.g. to 100% of the surface of the insertable area.

The covering may specifically form a continuous layer covering the surface section of the implantation needle. Thereby, the surface section may extend from the tip portion as described above. Specifically, the surface section corresponds to the tip portion and may include preferably at least 5%, preferably at least 10%, preferably at least 20%, preferably at least 30%, preferably at least 40%, preferably at least 50%, preferably at least 60%, preferably at least 70%, preferably at least 80%, preferably at least 90%, preferably at least 95% of the tip portion.

Specifically, the implantation needle may comprise at least one outer surface facing an outer environment of the implantation needle. Further, the implantation needle may comprise at least one inner surface facing an interior space of the implantation needle. The interior space may specifically refer to the lumen as described above. Further, the interior space may be fully or at least partially surrounded by the walls of the implantation needle. The covering may cover the outer surface of the implantation needle at least partially. The covering may cover preferably at least 5%, preferably at least 10%, preferably at least 20%, preferably at least 30%, preferably at least 40%, preferably at least 50%, preferably at least 60%, preferably at least 70%, preferably at least 80%, preferably at least 90%, preferably at least 95%, preferably 100% of the outer surface.

Further, as described above, the implantation needle may be a slotted needle having a slot extending in an axial direction. An intermediate area may be formed between at least one outer edge of the outer surface and at least one inner edge of the inner surface. The intermediate area may be covered with the covering at least partially, preferably fully.

Specifically, the implantation needle may be a slotted needle having a slot forming an angle of 10° to 80° to a longitudinal axis of the implantation needle and the slot comprises at least one cut surface extending from an edge point of the implantation needle. Thereby, the cut surface may be covered with the covering at least partially.

The implantation needle may comprise, as outlined above, the subcutaneously insertable element receiving portion and the tip portion, specifically at least one taper-shaped tip portion. The tip portion may be covered with the covering at least partially. Further, the subcutaneously insertable element receiving portion may be covered with the covering at least partially.

The tip portion may further comprise at least one adhesive coating. The adhesive coating may be configured to provide an adhesion and/or a bond between the covering and the implantation needle, specifically the surface section of the implantation needle as described above. The adhesive coating may be arranged between at least one surface of the implantation needle and the covering. Further, the subcutaneously insertable element receiving portion may comprise at least one non-stick coating. The non-stick coating may cover the subcutaneously insertable element receiving portion at least partially. Thus, when inserting the implantation needle, specifically the tip portion of the implantation needle, into the body tissue, a delamination of the covering from the implantation needle during inserting the subcutaneously insertable element is avoided. Further, when removing the implantation needle from the tissue, the covering is stripped off in an easy manner.

The term "remaining in the body tissue" as used herein is a broad term and is to be given its ordinary and customary meaning to a person of ordinary skill in the art and is not to be limited to a special or customized meaning. The term may refer to a staying of an element or a chemical within a body tissue specifically after a removal of an object from the body tissue. Specifically, the object and the element or chemical may be inserted into the body tissue at the same time, e.g. simultaneously, and the object may be removed while the element or the chemical is left behind and stays within the body tissue. Specifically, the pharmaceutical compound may be configured to be released via a mechanical stripping process during withdrawal of the implantation needle from the body tissue. Thus, a delamination of the pharmaceutical compound from a surface of the implantation needle may take place during removal of the implantation needle from the body tissue. Specifically, a bond between the adhesive bonding and the covering may be dissolved.

Further, additionally or alternatively, the pharmaceutical compound may be configured to be released via a dissolving process of the pharmaceutical compound. Thus, the pharmaceutical compound may be dissolved within the body tissue. Thereafter, the pharmaceutical compound may spread within the body tissue.

In a further aspect of the present invention, a kit for inserting a subcutaneously insertable element into a body tissue as defined in claim 11 is provided. The kit comprises at least one implantation needle as described above or as will further be described below in more detail. Further, the kit comprises at least one subcutaneously insertable element.

The term "kit" as used herein is a broad term and is to be given its ordinary and customary meaning to a person of ordinary skill in the art and is not to be limited to a special or customized meaning. The term may refer to a combination of components as specified, adapted for inserting the subcutaneously insertable element into the body tissue. Kits for subcutaneously inserting devices are in principle known to the skilled person, e.g. from WO 2016/012482 and references cited therein.

In the kit, the subcutaneously insertable element may be comprised in the subcutaneously insertable element receiving portion in order to be implanted into the body tissue, specifically via a translational movement, specifically via a common translational movement of the subcutaneously insertable element. Thus, specifically, the components of the kit, in particular the implantation needle and the subcutaneously insertable element may be comprised in a common casing, optionally comprising further components of the kit functionally connected thereto.

Specifically, all components required for subcutaneous insertion of the subcutaneously insertable element may be comprised in the casing of the kit. Thus, the kit may be a preassembled kit. Further, specifically, the kit may be a ready-to-use implantation kit. Thus, the kit may be a standalone system comprised of the components specified herein. Specifically, at least the implantation needle and the subcutaneously insertable element may be sterile.

The subcutaneously insertable element may be selected from the group consisting of: a sensor, preferably an analyte sensor, more preferably a continuous glucose monitoring sensor, most preferably a fully implantable continuous glucose monitoring sensor; an infusion cannula, preferably an insulin delivery cannula.

Further, the kit may comprise at least one discharging unit. The discharging unit may be configured for discharging the subcutaneously insertable element through the implantation needle. Exemplarily, the discharging unit may be a plunger. Moreover, the kit may comprise at least one of an actuator, a trigger which is configured for effecting the discharging of the subcutaneously insertable element.

The kit further may comprise at least one body patch adapted for attachment to a skin surface, such as to a skin surface of the patient or the user, in particular in cases where the subcutaneously insertable element is not fully implantable. In order to be attached to the skin surface, the body patch specifically may comprise one or more adhesive patches and/or plasters and/or other types of attachment elements for attachment of the body patch to the skin surface. The body patch may be adapted to be coupled to the kit during inserting the subcutaneously insertable element into the body tissue. Thus, the body patch may have one or more receptacles adapted for receiving a portion of the kit, in particular of the implantation needle and for holding the implantation needle.

In a further aspect of the present invention, a method of manufacturing an implantation needle as described above or as will further be described below in more detail is disclosed. The method for manufacturing an implantation needle comprises the method steps as given in the independent claims and as listed as follows. The method steps may be performed in the given order. However, other orders of the method steps are feasible. Further, one or more of the method steps may be performed in parallel and/or on a timely overlapping fashion. Further, one or more of the method steps may be performed repeatedly. Further, additional method steps may be present which are not listed.

The method comprises the following steps:
a) providing at least one needle element; and
b) covering at least one surface of the needle element with the pharmaceutical compound by at least one covering process such that the implantation needle is formed.

As further used herein, the term "manufacturing" may refer to an arbitrary process of producing or assembling an arbitrary device, specifically in a mechanical way. Specifically, the term manufacturing may refer to a value added production of a device for use. For the process of manufacturing machines, tools, chemical and biological processing or formulation may be applied. The manufacturing process may comprise a number of different manufacturing steps which may be performed in a given order. However, one or more of the manufacturing steps may be performed in parallel and/or on a timely overlapping fashion. Further, one or more of the manufacturing steps may be performed repeatedly. Exemplarily, the manufacturing process may begin with a creation of materials from which a device is made. These materials may then be modified through the manufacturing process to become the desired device. Further, exemplarily, the device may be assembled by combining several separate components. However, other embodiments may be feasible.

The covering process may specifically be or may comprise at least one coating process. Specifically, the covering process may be selected from the group consisting of: a dip coating process; a powder coating process; a spray coating process; a printing process, specifically an inkjet printing process; a lamination process; a dispensing process; a physical vapor deposition; a chemical vapor deposition; an electrospinning process; an electroplating process; a chemical surface functionalizing process. The chemical surface functionalizing process may specifically comprise any kind of a chemical coupling of a compound to a surface via a chemical reaction or a self-assembling process.

The proposed implantation needle and the proposed method of manufacturing an implantation needle provide many advantages over known devices and methods. Usually, the subcutaneously insertable element comprises the pharmaceutical compound such as the anti-inflammatory coating, specifically to increase the biocompatibility of the subcutaneously insertable element.

The pharmaceutical compound may specifically comprise the anti-inflammatory agent. The anti-inflammatory agent may at least partially remain in the body tissue after insertion of the subcutaneously insertable element and may be configured for reducing a physiological reaction of the subcutaneously insertable element. Covering the implantation needle instead of the subcutaneously insertable element makes the manufacturing of the subcutaneously insertable element easier.

During inserting of the subcutaneously insertable element into the body tissue, the anti-inflammatory agent may be released. Thus, during the so-called acute inflammatory response an inflammatory reaction is inhibited. As specifically the first few hours after a physical injury play an important role for a development of an inflammatory reaction, a severity of the inflammatory reaction and/or of the encapsulation may be inhibited via the implantation needle according to the present invention. Thus, exemplarily, a wearing time of the continuous monitoring sensor may be extended. Exemplarily, a wearing time of the subcutaneously insertable element within the body tissue of 14 days or even more may be achieved. Due to differences in a solubility of different pharmaceutical compounds, a short time effect and a deferred long-term effect of an inflammation inhibition may be achieved.

Summarizing and without excluding further possible embodiments, the following embodiments may be envisaged:

### Short description of the Figures

Further optional features and embodiments will be disclosed in more detail in the subsequent description of embodiments, preferably in conjunction with the dependent claims. Therein, the respective optional features may be realized in an isolated fashion as well as in any arbitrary feasible combination, as the skilled person will realize. The embodiments are schematically depicted in the Figures. Therein, identical reference numbers in these Figures refer to identical or functionally comparable elements.

In the Figures:
- Figure 1: shows an exemplary embodiment of a kit according to the present invention in a top view; and
- Figure 2: shows an exemplary embodiment of an implantation needle according to the present invention in a cross-sectional view.

### Detailed description of the embodiments

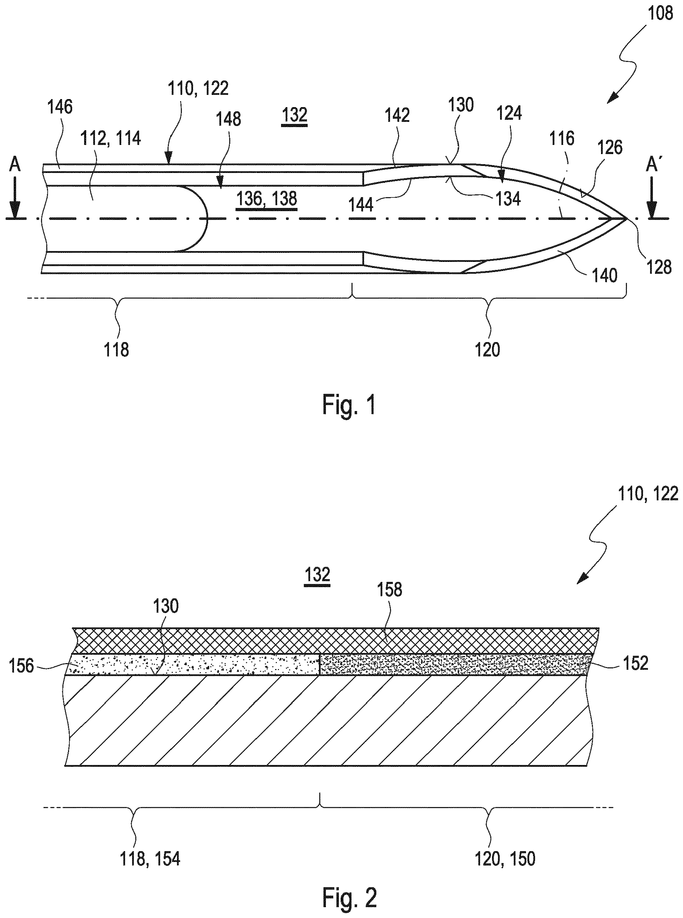

Figure 1 shows an exemplary embodiment of a kit 108 for inserting a subcutaneously insertable element 112 into a body tissue. The kit 108 comprises an implantation needle 110 and a subcutaneously insertable element 112.

The implantation needle 110 may host the subcutaneously insertable element 112. The subcutaneously insertable element 112 may exemplarily be an analyte sensor 114. The analyte sensor 114 may extend along a longitudinal axis A - A' (denoted by reference number 116) in Figure 1. The subcutaneously insertable element 112 may be received within the implantation needle 110.

Specifically, the implantation needle 110 may have an insertable element receiving portion 118 and a tip portion 120. Further, the implantation needle 110 may be a slotted needle 122. The slotted needle 122 may have a first slot 124 forming an angle of 10° to 80° to the longitudinal axis 116 of the implantation needle 110. The first slot 124 comprises the at least one cut surface 126 extending from an edge point 128 of the implantation needle 110. Specifically, the implantation needle 110 may comprise at least one outer surface 130 facing an outer environment 132 of the implantation needle 110 and at least one inner surface 134 facing an interior space 136, specifically a lumen 138, of the implantation needle 110. The interior space 136 may specifically be at least partially enclosed by walls 146 of the implantation needle 110. The cut surface 126 specifically may correspond to an intermediate area 140 formed between at least one outer edge 142 of the outer surface 130 and at least one inner edge 144 of the inner surface 134. Further the implantation needle 110 may have a second slot 148 extending along the longitudinal axis 116.

Moreover, the implantation needle 110 comprises at least one covering (not illustrated in Figure 1). The covering comprises at least one pharmaceutical compound. The covering may exemplarily cover the tip portion 120, specifically the outer surface 130 of the tip portion 120 and/or the cut surface 126. Still, however, a different distribution of the covering onto the implantation needle 110 is feasible.

Figure 2 shows an exemplary embodiment of an implantation needle 110 in a cross-sectional view. The implantation needle 110 corresponds at least in wide parts to the implantation needle 110 as illustrated in Figure 1. Thus, reference can be made to the description of Figure 1 above.

As outlined above, the implantation needle may comprise the at least one outer surface 130 facing the outer environment 132 of the implantation needle 110. One first portion 150 of the outer surface 130 may be covered with at least one adhesive coating 152. One second portion 154 may be covered with at least one non-stick coating 156. Specifically, the first portion 150 may correspond to the tip portion 120 as described above and the second portion 154 may correspond to the insertable element receiving portion 118 as described above. However, other embodiments may be feasible.

Further, the implantation needle 110 comprises the at least one covering, as denoted with reference number 158 in Figure 2. The covering 158 comprises at least one pharmaceutical compound. The adhesive coating 152 may be arranged between the outer surface 130 and the covering 158. Specifically, the adhesive coating 152 may be configured to provide a bonding between the implantation needle 110 and the covering 158. Specifically, the coating 158 may be arranged on top of the adhesive coating 152 and the non-stick coating 156. However, also other embodiments may be feasible.

### List of reference numbers

- 108: Kit
- 110: implantation needle
- 112: subcutaneously insertable element
- 114: analyte sensor
- 116: longitudinal axis
- 118: insertable element receiving portions
- 120: tip portion
- 122: slotted needle
- 124: first slot
- 126: cut surface
- 128: edge point
- 130: outer surface
- 132: outer environment
- 134: inner surface
- 136: interior space
- 138: Lumen
- 140: intermediate space
- 142: outer edge
- 144: inner edge
- 146: Wall
- 148: second slot
- 150: first portion
- 152: adhesive coating
- 154: second portion
- 156: non-stick coating
- 158: covering

## Claims

1. An implantation needle (110) for inserting a subcutaneously insertable element (112) into a body tissue, wherein the implantation needle (110) comprises at least one covering (158) comprising at least one pharmaceutical compound, wherein the pharmaceutical compound is configured to remain at least partially in the body tissue after insertion of the subcutaneously insertable element (112), wherein the pharmaceutical compound is configured to be released via a mechanical stripping process during withdrawal of the implantation needle (110) from the body tissue or via a dissolving process of the pharmaceutical compound.

2. The implantation needle (110) according to claim 1, wherein the pharmaceutical compound is selected from the group consisting of: an immunosuppressive, an anti-allergic, an anti-inflammatory agent.

3. The implantation needle (110) according to any one of claims 1 to 2, wherein the pharmaceutical compound is selected from the group consisting of: dexamethasone, a derivative of dexamethasone, betamethasone, a derivative of betamethasone, wherein the derivative of Betamethasone or Dexamethasone is selected from the group consisting of 17-Oxo Betamethasone, Betamethasone hydrochloride, Betamethasone 21-acetate, Betamethasone 17-propionate, Betamethasone 21 propionate, Betamethasone phosphate, Betamethasone 17-valerate, Betamethasone 21-valerate, Betamethasone 17-benzoate, Betamethasone 17,21,-dipropionate, Betamethasone acibutate, Betamethasone sodium phosphate, Betamethasone butyrate propionate, Betamethasone tripropionate, 3-Hydroxy dexamethasone, 6 Hydroxy dexamethasone, dexamethasone 17-acetate, dexamethasone 17-carboxamide, dexamethasone 17-propionate, dexamethasone 21-valerate, dexamethasone 21-acetate, dexamethasone 21-beta-D-glucoside, dexamethasone 21-linolate, dexamethasone 21-mesylate, dexamethasone 21-O-b-D-galactopyranose, dexamethasone 21-palmitate, dexamethasone 21-propionate, dexamethasone 21-sodium hydrogen phosphate, dexamethasone 21-sulfate, dexamethasone 21-tributylacetate, dexamethasone acefurate, dexamethasone 17-carboxylic acid, dexamethasone beta-D-glucuronide, dexamethasone dipropionate, Dexamethasone hemisuccinate, dexamethasone laurate, dexamethasone phosphate, dexamethasone sodium phosphate, dexamethasone tridecylate, dexamethasone valerate, dexamethasone 21-isonicotinate; in an embodiment is selected from the list consisting of Betamethasone 21-acetate, Betamethasone 17-propionate, Betamethasone 21 propionate, Betamethasone 17-valerate, Betamethasone 21-valerate, Betamethasone butyrate propionate, Betamethasone tripropionate, dexamethasone 17-acetate, dexamethasone 17-carboxamide, dexamethasone 17-propionate, dexamethasone 21-valerate, dexamethasone 21-acetate, dexamethasone 21-linolate, dexamethasone 21-mesylate, dexamethasone 21-palmitate, dexamethasone 21-propionate, dexamethasone 21-tribu-tylacetate, dexamethasone acefurate, dexamethasone dipropionate, dexamethasone laurate, dexamethasone tridecylate, dexamethasone valerate.

4. The implantation needle (110) according to any one of claims 1 to 3, wherein the pharmaceutical compound comprises at least one compound which is configured to improve a tolerance of the subcutaneously insertable element (112).

5. The implantation needle (110) according to any one of claims 1 to 4, wherein the pharmaceutical compound is configured to reduce at least one of an immune rejection, a scarring, a growth of infectious agents.

6. The implantation needle (110) according to any one of claims 1 to 5, wherein the covering (158) comprises at least one excipient.

7. The implantation needle (110) according to claim 6, wherein the excipient is selected from the group consisting of: a polylactide, a polyglycolide, a polydioxanone.

8. The implantation needle (110) according to any one of claims 1 to 7, wherein the implantation needle (110) comprises at least one outer surface (130) facing an outer environment (132) of the implantation needle (110) and at least one inner surface (134) facing an interior space (136) of the implantation needle (110), wherein the covering (158) covers the outer surface (130) of the implantation needle (110) at least partially.

9. The implantation needle (110) according to any one of claims 1 to 8, wherein the implantation needle (110) comprises at least one subcutaneously insertable element receiving portion (118) and at least one tip portion (120), wherein the tip portion (120) is covered with the covering (158) at least partially.

10. The implantation needle (110) according to claim 9, wherein the tip portion (120) further comprises at least one adhesive coating (152), wherein the adhesive coating (152) is arranged between at least one surface of the implantation needle (110) and the covering (158).

11. A kit (108) for inserting a subcutaneously insertable element (112) into a body tissue, wherein the kit (108) comprises:
• at least one implantation needle (110) according to any one of claims 1 to 10; and
• at least one subcutaneously insertable element (112).

12. A method of manufacturing an implantation needle (110) according to any one of claims 1 to 10, wherein the method comprises the following steps:
a) providing at least one needle element; and
b) covering at least one surface of the needle element with the pharmaceutical compound by at least one covering process such that the implantation needle (110) is formed.

13. The method according to claim 12, wherein the covering process is selected from the group consisting of: a dip coating process; a powder coating process; a spray coating process; a printing process; a lamination process; a dispensing process; a physical vapor deposition; a chemical vapor deposition; an electrospinning process; an electroplating process; a chemical surface functionalizing process.

## Patentansprüche

1. Implantationsnadel (110) zum Einsetzen eines subkutan einführbaren Elements (112) in ein Körpergewebe, wobei die Implantationsnadel (110) mindestens eine Umhüllung (158) umfasst, die mindestens eine pharmazeutische Verbindung umfasst, wobei die pharmazeutische Verbindung dafür ausgebildet ist, nach dem Einsetzen des subkutan einführbaren Elements (112) mindestens teilweise im Körpergewebe zu verbleiben, wobei die pharmazeutische Verbindung dafür ausgebildet ist, mittels eines mechanischen Ablöseprozesses während des Herausziehens der Implantationsnadel (110) aus dem Körpergewebe oder mittels eines Auflösungsprozesses der pharmazeutischen Verbindung freigesetzt zu werden.

2. Implantationsnadel (110) nach Anspruch 1, wobei die pharmazeutische Verbindung aus der Gruppe ausgewählt ist, die aus Folgendem besteht: einem Immunsuppressivum, einem Antiallergikum, einem Entzündungshemmer.

3. Implantationsnadel (110) nach einem der Ansprüche 1 bis 2, wobei die pharmazeutische Verbindung aus der Gruppe ausgewählt ist, die aus Folgendem besteht: Dexamethason, einem Derivat von Dexamethason, Betamethason, einem Derivat von Betamethason, wobei das Derivat von Betamethason oder Dexamethason aus der Gruppe ausgewählt ist, die aus 17-Oxo-Betamethason, Betamethasonhydrochlorid, Betamethason-21-acetat, Betamethason-17-propionat, Betamethason-21-propionat, Betamethasonphosphat, Betamethason-17-valerat, Betamethason-21-valerat, Betamethason-17-benzoat, Betamethason-17,21-dipropionat, Betamethasonacibutat, Betamethasonnatriumphosphat, Betamethasonbutyratpropionat, Betamethasontripropionat, 3-Hydroxydexamethason, 6-Hydroxydexamethason, Dexamethason-17-acetat, Dexamethason-17-carboxamid, Dexamethason-17-propionat, Dexamethason-21 -valerat, Dexamethason-21-acetat, Dexamethason-21-beta-D-glucosid, Dexamethason-21-linolat, Dexamethason-21-mesylat, Dexamethason-21-O-b-D-galactopyranose, Dexamethason-21-palmitat, Dexamethason-21-propionat, Dexamethason-21-natriumhydrogenphosphat, Dexamethason-21-sulfat, Dexamethason-21-tributylacetat, Dexamethasonacefurat, Dexamethason-17-carbonsäure, Dexamethason-beta-D-glucuronid, Dexamethasondipropionat, Dexamethasonhemisuccinat, Dexamethasonlaurat, Dexamethasonphosphat, Dexamethasonnatriumphosphat, Dexamethasontridecylat, Dexamethasonvalerat, Dexamethason-21-isonicotinat besteht; in einer Ausführungsform aus der Liste ausgewählt ist, die aus Betamethason-21-acetat, Betamethason-17-propionat, Betamethason-21-propionat, Betamethason-17-valerat, Betamethason-21-valerat, Betamethasonbutyratpropionat, Betamethasontripropionat, Dexamethason-17-acetat, Dexamethason-17-carboxamid, Dexamethason-17-propionat, Dexamethason-21-valerat, Dexamethason-21-acetat, Dexamethason-21-linolat, Dexamethason-21-mesylat, Dexamethason-21-palmitat, Dexamethason-21-propionat, Dexamethason-21-tributylacetat, Dexamethasonacefurat, Dexamethasondipropionat, Dexamethasonlaurat, Dexamethasontridecylat, Dexamethasonvalerat besteht.

4. Implantationsnadel (110) nach einem der Ansprüche 1 bis 3, wobei die pharmazeutische Verbindung mindestens eine Verbindung umfasst, die dafür ausgebildet ist, eine Toleranz des subkutan einführbaren Elements (112) zu verbessern.

5. Implantationsnadel (110) nach einem der Ansprüche 1 bis 4, wobei die pharmazeutische Verbindung dafür ausgebildet ist, mindestens eines von einer Immunabstoßung, einer Narbenbildung, einem Wachstum von Infektionskeimen zu reduzieren.

6. Implantationsnadel (110) nach einem der Ansprüche 1 bis 5, wobei die Umhüllung (158) mindestens einen Hilfsstoff umfasst.

7. Implantationsnadel (110) nach Anspruch 6, wobei der Hilfsstoff aus der Gruppe ausgewählt ist, die aus Folgendem besteht: einem Polylactid, einem Polyglycolid, einem Polydioxanon.

8. Implantationsnadel (110) nach einem der Ansprüche 1 bis 7, wobei die Implantationsnadel (110) mindestens eine Außenfläche (130), die einem äußeren Umfeld (132) der Implantationsnadel (110) zugewandt ist, und mindestens eine Innenfläche (134), die einem Innenraum (136) der Implantationsnadel (110) zugewandt ist, umfasst, wobei die Umhüllung (158) die Außenfläche (130) der Implantationsnadel (110) mindestens teilweise umhüllt.

9. Implantationsnadel (110) nach einem der Ansprüche 1 bis 8, wobei die Implantationsnadel (110) mindestens einen Aufnahmeabschnitt (118) für ein subkutan einführbares Element und mindestens einen Spitzenabschnitt (120) umfasst, wobei der Spitzenabschnitt (120) mindestens teilweise mit der Umhüllung (158) umhüllt ist.

10. Implantationsnadel (110) nach Anspruch 9, wobei der Spitzenabschnitt (120) ferner mindestens eine Haftbeschichtung (152) umfasst, wobei die Haftbeschichtung (152) zwischen mindestens einer Fläche der Implantationsnadel (110) und der Umhüllung (158) angeordnet ist.

11. Kit (108) zum Einsetzen eines subkutan einführbaren Elements (112) in ein Körpergewebe, wobei der Kit (108) Folgendes umfasst:
• mindestens eine Implantationsnadel (110) nach einem der Ansprüche 1 bis 10; und
• mindestens ein subkutan einführbares Element (112).

12. Verfahren zum Herstellen einer Implantationsnadel (110) nach einem der Ansprüche 1 bis 10, wobei das Verfahren die folgenden Schritte umfasst:
a) Bereitstellen mindestens eines Nadelelements; und
b) Umhüllen mindestens einer Fläche des Nadelelements mit der pharmazeutischen Verbindung mittels mindestens eines Umhüllungsprozesses, so dass die Implantationsnadel (110) gebildet wird.

13. Verfahren nach Anspruch 12, wobei der Umhüllungsprozess aus der Gruppe ausgewählt ist, die aus Folgendem besteht: einem Tauchbeschichtungsprozess; einem Pulverbeschichtungsprozess; einem Sprühbeschichtungsprozess; einem Bedruckprozess; einem Laminierprozess; einem Dispensierprozess; einer physikalischen Dampfbeschichtung; einer chemischen Dampfbeschichtung; einem Elektrospinnprozess; einem Elektroplattierungsprozess; einem chemischen Oberflächenfunktionalisierungsprozess.

## Revendications

1. Aiguille d'implantation (110) pour insertion d'un élément insérable de manière sous-cutanée (112) dans un tissu corporel, dans lequel l'aiguille d'implantation (110) comprend au moins un enrobage (158) comprenant au moins un composé pharmaceutique, dans laquelle le composé pharmaceutique est conçu pour rester au moins partiellement dans le tissu corporel après insertion de l'élément insérable de manière sous-cutanée (112), dans laquelle le composé pharmaceutique est conçu pour être libéré par le biais d'un procédé de séparation mécanique pendant le retrait de l'aiguille d'implantation (110) du tissu corporel ou par le biais d'un procédé de dissolution du composé pharmaceutique.

2. Aiguille d'implantation (110) selon la revendication 1, dans laquelle le composé pharmaceutique est choisi dans le groupe constitué par : un immunosuppresseur, un anti-allergique, un agent anti-inflammatoire.

3. Aiguille d'implantation (110) selon l'une quelconque des revendications 1 à 2, dans lequel le composé pharmaceutique est choisi dans le groupe constitué par : la dexaméthasone, un dérivé de la dexaméthasone, la bétaméthasone, un dérivé de la bétaméthasone, dans laquelle le dérivé de la bétaméthasone ou de la dexaméthasone est choisi dans le groupe constitué par la 17-oxobétaméthasone, le chlorhydrate de bétaméthasone, le 21-acétate de bétaméthasone, le 17-propionate de bétaméthasone, le 21-propionate de bétaméthasone, le phosphate de bétaméthasone, le 17-valérate de bétaméthasone, le 21-valérate de bétaméthasone, le 17-benzoate de bétaméthasone, le 17,21-dipropionate de bétaméthasone, l'acibutate de bétaméthasone, le phosphate sodique de bétaméthasone, le butyrate-propionate de bétaméthasone, le tripropionate de bétaméthasone, la 3-hydroxydexaméthasone, la 6-hydroxydexaméthasone, le 17-acétate de dexaméthasone, le dexaméthasone-17-carboxamide, le 17-propionate de dexaméthasone, le 21-valérate de dexaméthasone, le 21-acétate de dexaméthasone, le dexaméthasone-21-bêta-D-glucoside, le 21-linolate de dexaméthasone, le 21-mésylate de dexaméthasone, le dexaméthasone-21-O-b-D-galactopyranose, le 21-palmitate de dexaméthasone, le 21-propionate de dexaméthasone, le 21-hydrogénophosphate sodique de dexaméthasone, le 21-sulfate de dexaméthasone, le 21-tributylacétate de dexaméthasone, l'acéfurate de dexaméthasone, l'acide dexaméthasone-17-carboxylique, le dexaméthasone-bêta-D-glucuronide, le dipropionate de dexaméthasone, l'hémisuccinate de dexaméthasone, le laurate de dexaméthasone, le phosphate de dexaméthasone, le phosphate sodique de dexaméthasone, le tridécylate de dexaméthasone, le valérate de dexaméthasone, le 21-isonicotinate de dexaméthasone ; dans un mode de réalisation est choisi dans la liste constituée par le 21-acétate de bétaméthasone, le 17-propionate de bétaméthasone, le 21-propionate de bétaméthasone, le 17-valérate de bétaméthasone, le 21-valérate de bétaméthasone, le butyrate-propionate de bétaméthasone, le tripropionate de bétaméthasone, le 17-acétate de dexaméthasone, le dexaméthasone-17-carboxamide, le 17-propionate de dexaméthasone, le 21-valérate de dexaméthasone, le 21-acétate de dexaméthasone, le 21-linolate de dexaméthasone, le 21-mésylate de dexaméthasone, le 21-palmitate de dexaméthasone, le 21-propionate de dexaméthasone, le 21-tributylacétate de dexaméthasone, l'acéfurate de dexaméthasone, le dipropionate de dexaméthasone, le laurate de dexaméthasone, le tridécylate de dexaméthasone, le valérate de dexaméthasone.

4. Aiguille d'implantation (110) selon l'une quelconque des revendications 1 à 3, dans lequel le composé pharmaceutique comprend au moins un composé qui est conçu pour améliorer une tolérance de l'élément insérable de manière sous-cutanée (112).

5. Aiguille d'implantation (110) selon l'une quelconque des revendications 1 à 4, dans lequel le composé pharmaceutique est conçu pour réduire au moins un parmi un rejet immunitaire, une cicatrice, une croissance d'agents infectieux.

6. Aiguille d'implantation (110) selon l'une quelconque des revendications 1 à 5, dans laquelle l'enrobage (158) comprend au moins un excipient.

7. Aiguille d'implantation (110) selon la revendication 6, dans laquelle l'excipient est choisi dans le groupe constitué par : un polylactide, un polyglycolide, une polydioxanone.

8. Aiguille d'implantation (110) selon l'une quelconque des revendications 1 à 7, dans laquelle l'aiguille d'implantation (110) comprend au moins une surface extérieure (130) faisant face à un environnement extérieur (132) de l'aiguille d'implantation (110) et au moins une surface intérieure (134) faisant face à un espace intérieur (136) de l'aiguille d'implantation (110), dans laquelle l'enrobage (158) enrobe la surface extérieure (130) de l'aiguille d'implantation (110) au moins partiellement.

9. Aiguille d'implantation (110) selon l'une quelconque des revendications 1 à 8, dans laquelle l'aiguille d'implantation (110) comprend au moins une partie de réception (118) d'élément insérable de manière sous-cutanée et au moins une partie de pointe (120), dans laquelle la partie de pointe (120) est enrobée avec l'enrobage (158) au moins partiellement.

10. Aiguille d'implantation (110) selon la revendication 9, dans laquelle la partie de pointe (120) comprend en outre au moins un revêtement adhésif (152), dans laquelle le revêtement adhésif (152) est agencé entre au moins une surface de l'aiguille d'implantation (110) et l'enrobage (158).

11. Kit (108) pour insertion d'un élément insérable de manière sous-cutanée (112) dans un tissu corporel, dans lequel le kit (108) comprend :
• au moins une aiguille d'implantation (110) selon l'une quelconque des revendications 1 à 10 ; et
• au moins un élément insérable de manière sous-cutanée (112).

12. Procédé de fabrication d'une aiguille d'implantation (110) selon l'une quelconque des revendications 1 à 10, dans lequel le procédé comprend les étapes suivantes :
a) fourniture d'au moins un élément d'aiguille ; et
b) enrobage d'au moins une surface de l'élément d'aiguille avec le composé pharmaceutique par au moins un procédé d'enrobage de telle sorte que l'aiguille d'implantation (110) est formée.

13. Procédé selon la revendication 12, dans lequel le procédé d'enrobage est choisi dans le groupe constitué de : un procédé de revêtement par immersion ; un procédé de revêtement de poudre ; un procédé de revêtement par atomisation ; un procédé d'impression ; un procédé de laminage ; un procédé de distribution ; un dépôt physique en phase vapeur ; un dépôt chimique en phase vapeur; un procédé d'électrofilage; un procédé d'électrodéposition ; un procédé de fonctionnalisation de surface chimique.
